Europäisches Patentamt

European Patent Office

Office européen des brevets

(11) Publication number: **0 181 596**
**B1**

## (12) EUROPEAN PATENT SPECIFICATION

(45) Date of publication of patent specification: **22.03.89**

(21) Application number: **85114064.0**

(22) Date of filing: **05.11.85**

(51) Int. Cl.⁴: **A 61 K 37/54,** C 12 N 11/02,
C 12 N 9/72 // A61K37/54

(54) Process for preparing a urokinase complex.

(30) Priority: **05.11.84 JP 232925/84**

(43) Date of publication of application:
**21.05.86 Bulletin 86/21**

(45) Publication of the grant of the patent:
**22.03.89 Bulletin 89/12**

(84) Designated Contracting States:
**BE CH DE FR GB LI**

(56) References cited:
**GB-A-2 021 412**
**US-A-4 029 767**

**CHEMICAL ABSTRACTS, vol. 85, no. 15, 11th
October 1976, page 233, abstract no. 105924n,
Columbus, Ohio, US; I. CLEMMENSEN et al.:
"Inhibition of urokinase by purified human
alpha1-antitrypsin", & PROTIDES BIOL.
FLUIDS, PROC. COLLOQ. 1975, (Publ. 1976), 23,
39-42**

(73) Proprietor: **TERUMO KABUSHIKI KAISHA
trading as TERUMO CORPORATION
44-1, 2-chome, Hatagaya Shibuya-Ku
Tokyo 151 (JP)**

(72) Inventor: **Taguchi, Shigeru
17-6, 3-chome Tsurumaki
Tama-shi Tokyo (JP)**
Inventor: **Maruyama, Teppei
36-3, 3-chome Nakaizumi
Komae-shi Tokyo (JP)**
Inventor: **Nakagame, Fujio
11-27, 1-chome Kasuya
Setagaya-ku Tokyo (JP)**
Inventor: **Honda, Haruo
5, 3-chome Saiwai-cho Saiwai-ku
Kawasaki-shi Kanagawa (JP)**

(74) Representative: **Henkel, Feiler, Hänzel & Partner
Möhlstrasse 37
D-8000 München 80 (DE)**

(56) References cited:

CHEMICAL ABSTRACTS, vol. 92, no. 5, 4th February 1980, page 509, abstract no. 38700r, Columbus, Ohio, US; H. SUMI et al.: "Changes in the molecular weight of urokinase by reduction and in plasma", & IGAKU TO SEIBUTSUGAKU 1979, 98(1), 47-51

CHEMICAL ABSTRACTS, vol. 99, no. 19, 7th November 1983, page 418, abstract no. 156039z, Columbus, Ohio, US, K.E. WALLER et al.: "Complex formation and inhibition of urokinase by blood plasma proteins", & BIOCHEM. J. 1983, 215(1), 123-31

PROC. 12TH CONGR. INT. SOC. BLOOD. TRANSF., no. 38, part II, 1969, pages 502-503,

Basel, CH; S.M. MARTYNOV et al.: "Urokinase in a complex with plasmin and as an independent factor for treatment of a thrombo-embolism"

**Description**

Background of the invention

This invention relates to a novel process for preparing a urokinase complex by binding urokinase with blood components.

Urokinase is one of plasminogen activators found in urine, which is biosynthesized in kidney and excreted in urine. Urokinase converts plasminogen to plasmin by reacting specifically with plasminogen and breaking arginyl-valyl bonds thereof. Urokinase has found a wide variety of applications, for example, as a thrombolytic agent for thrombolytic therapy of cerabral thrombosis, cardiac infarction, plumonary embolism, etc., and for combined therapy with an anti-tumor agent such as Mitomycin C (trade name).

Urokinase is administered by intravenous injection or infusion because it is little absorbed through the gastrointestinal tract. The urokinase, after intravenously administered, is rapidly inhibited by inhibitors in blood such as $\alpha_2$-plasmin inhibitor and the like. The half time of urokinase in blood is as short as about 15 minutes. Thus the effectiveness of urokinase disappears so fast.

Further, urokinase has a low binding affinity to fibrin and fibrin clot or thrombus and sustains its fibrinolytic activity only for a short time when administered in vivo. The concentration of urokinase in blood must be increased above a certain threshold to achieve satisfactory fibrinolytic activity. This, in turn, requires to administer a large amount of urokinase.

Summary of the invention

An object of the present invention is to provide an improved method for preparing a novel urokinase complex which has a high affinity to fibrin and maintains its fibrinolytic activity in vivo for a prolonged period.

According to the process of the present invention, a novel urokinase complex is prepared by mixing urokinas with wholse blood, blood serum or blood plasma allowing the mixture to react with the serum protein or plasma protein to form a urokinase complex fraction and separating and purifying said urokinase complex fraction having a molecular weight of 80,000 to 120,000 from the reaction mixture. There is obtained the urokinase complex in essentially pure form ready for medical use.

The blood component to be bound to urokinase may preferably be blood serum or plasma although whole blood may also be employed in the practice of the present invention. Complexes of urokinase and purified blood components, either protein or polysaccharide are disclosed in the Chem. Abstracts. V. 85, N 15, October 11, 1976, ref 105924 N or in the US—A—4029767.

Brief description of the drawings

The invention will be more fully understood by reading the following description taken in conjunction with the accompanying drawings, in which:

Fig. 1 illustrates the half time in blood of the present urokinase complex and urokinase when they are intravenously injected to rats;

Fig. 2 illustrates the half time in blood of the present urokinase complex and urokinase when they are intravenously injected to rabbits; and

Fig. 3 illustrates the half time in blood of the present urokinase complex and urokinase when they are intravenously injected to beagles.

Detailed description of the invention

Although urokinase has been administered by injection, development of a urokinase preparation suitable for oral administration is desired to eliminate the trouble of the doctor and relieve the pain of the patient. We found that urokinase sustains its effectiveness for a longer period when orally administered than when intravenously administered.

To determine the cause for this prolonged effectiveness, the behavior of orally administered urokinase in blood was investigated by zymography which is slab gel electrophoresis combined with fibrin plate method. We have found that the urokinase, when absorbed, is converted into a macromolecule having a molecular weight of about 94,000, which can stay in blood for a prolonged period, maintaining the effectiveness.

We have also found that such a macromolecule may also be produced in vitro by mixing urokinase with whole blood, plasma or serum followed by incubation at an appropriate temperature. The present invention is based on these findings.

The urokinase complex of the present invention was not detected in blood just collected from a living body to which urokinase had been intravenously injected. It is believed that when urokinase is administered to a living body, a quantity of urokinase inhibitors such as $\alpha_2$-plasmin inhibitor is produced in blood to inactivate the urokinase prior to the formation of a urokinase complex.

In contrast, the collected blood which is not under the physiological regulation of the living body allows urokinase to be reactive to form the urokinase complex of the present invention.

Any purified urokinase for medical use may be employed in the process of the invention irrespective of whether it is isolated from human urine or kidney tissue culture or synthesized by genetic engineering. The urokinase may preferably have a molecular weight from 30,000 to 60,000. Single chain urokinase with a much higher binding affinity to fibrin than conventional urokinase and tissue plasminogen activator (TPA) may also be employed in the process of the invention.

Whole blood, plasma or serum may be added for supplying the blood component although the use of plasma or serum is preferred.

The phrase "a blood component other than blood corpuscles" used herein means a remainder of blood from which at least erythrocyte, leucocyte and platelet are removed, which is typically available as serum and plasma. The presence of the corpscules is not critical in the practice of the present invention. Any remainders of blood from which an additional blood component or components are removed may also be employed in the present invention. The phrase "at least a blood component" is used to encompass the use of whole blood.

The term "urokinase complex" used herein means that urokinase is bound to a certain blood component through reaction with a protein, albumin and/or globulin fraction in plasma or serum, forming an integral complex.

According to the present invention, the urokinase complex is prepared by mixing urokinase with the blood component supplied as whole blood, plasma, serum or the like to form a reaction mixture. The reaction mixture is incubated at an appropriate temperature in the range from 4 to 40°C for several minutes, preferably for 15 to 30 minutes. During thermostatic incubation, the reaction mixture may be either stirred or allowed to stand. When the reaction is completed, the urokinase complex is isolated and purified by any well-known conventional method such as gel filtration, affinity chromatography, electrophoresis, ultrafiltration, or by any combination of these methods.

The composition of the purified urokinase complex is not definitely determined, but it is a novel macromolecular complex having a molecular weight of 80,000 to 120,000.

The urokinase complex produced by the present process may be frozen and stored at −20°C or below, or it may be freeze-dried for storage.

The novel urokinase complex of the invention or thrombolytic agents containing the same may be employed in the thrombolytic therapy for cerabral thrombosis, cardiac infarction, plumonary embolism or the like by way of intravenous injection or infusion, or oral administration.

The urokinase complex thus obtained may be prepared into a thrombolytic agent in which an effective amount of urokinase complex is contained. Any conventional known vehicles used in commercial urokinase preparations such as albumin, gelatin or the like may be added to keep the urokinase complex stable in the thrombolytic agent.

Examples

Examples of the present invention are presented below together with experiments. These examples are to be construed as illustrating the present invention and not limiting the present invention.

Example 1

0.1 ml urokinase (molecular weight 54,000, 100,000 IU/ml, phosphate buffer, pH 7.2) was mixed with 1 ml rat plasma and incubated at 20°C for 30 min. for reaction. When the reaction was completed, the reaction solution was gel filtered over a Sephadex® G-100 column (2.6 cm diameter×100 cm) which had been equilibrated at pH 7.5 with phosphate buffer containing 0.17 M sodium chloride. The column was operated with the buffer for elution. The effluent was passed to a fraction collector, and the resulting fractions assayed for fibrinolytic activity on fibrin plates. Among them, there were observed those fractions containing a urokinase complex and unreacted urokinase, respectively. The molecular weight of the urokinase complex was determined by gel filtration to be approximately 93,000. The resulting urokinase complex was frozen and stored at −80°C. The urokinase complex had a total activity of 6,300 IU when assayed on fibrin plate.

Example 2

0.1 ml urokinase (molecular weight 54,000, 20,000 IU/ml, phosphate buffer, pH 7.2) was mixed with 1 ml rabbit serum and incubated at 30°C for 15 min. for reaction. When the reaction was completed, the reaction solution was mixed with an equal amount of 0.5 M Tris buffer containing 10% sodium dodecyl sulfate (SDS) and 50% glycerin, and subjected to SDS-polyacrylamide slab gel electrophoresis (10% gel, size 14×14×0.2 cm) by using a 0.025 M Tris-0.19 M glycine buffer containing 0.1% SDS at pH 8.3 and operating at 8 mA, 4°C for approximately 16 hours. A strip of 5 mm wide was cut from either end of the gel in a direction parallel to the migration, and immersed in 25% Triton®X-100 for 1 hour followed by rinsing to remove the SDS. Filter paper was allowed onto the gel strips to remove water therefrom. The strips were placed on a fibrin plate and incubated at 37°C for about 15 hours to determine fibrinolytically active regions. The strips and the central portion of the gel which had been stored at 4°C were then placed side by side to identify the fibrinolytically active regions on the gel. That portion of the gel corresponding to the high molecular weight active region (containing urokinase complex) was cut out and disintegrated to pieces, which were placed in a protein recovering apparatus (AE-3590 Max-yield-GP manufactured by Atoh K. K.). Using a recovering buffer prepared by diluting the above-mentioned buffer to a ten-fold volume, electrophoresis was conducted for 4 hours at 4W and 4°C to recover the urokinase complex. The molecular weight of the urokinase complex was determined by gel electrophoresis to be approximately 89,000. The resulting urokinase complex was frozen at −80°C, freeze-dried, and stored. The urokinase complex had a total activity of 7,500 IU when assayed on fibrin plate.

Example 3

0.1 ml urokinase (molecular weight 54,000, 500,000 IU/ml, phosphate buffer, pH 7.2) was mixed with 1 ml whole blood of a beagle having citrate solution added thereto, and incubated at 25°C for 20 min. for reaction. When the reaction was completed, the reaction solution was centrifuged at 4°C, 3,000 rpm to separate the plasma. The resulting plasma was mixed with an equal amount of 0.5 M Tris buffer containing 10% SDS and 50% glycerin. The reaction solution was placed in a preparative gel electrophoresis system (manufactured by Maruzen Petroleum Biochemical K. K.). Using 0.025 M Tris-0.19 M glycine buffer containing 0.1% SDS at pH 8.3, electrophoresis was conducted for about 15 hours at 4 mA and 4°C. The gel tube was filled with SDS-polyacrylamide gel (10% gel). During the electrophoresis, the Tris-glycine buffer was introduced into the electrophoresis system at a flow rate of 2 ml/hour. The effluents were divided into 1 ml fractions by means of a fraction collector. Aliquot of each fraction was subjected to slab electrophoresis and assayed by the fibrin-plate method as in Example 2 to identify the fractions of urokinase and urokinase complex. The urokinase complex fraction was dialyzed with water in a dialyzing tube to remove the SDS. The molecular weight in the urokinase complex was determined by electrophoresis to be approximately 93,000. The resulting urokinase complex was frozen at −80°C, freeze-dried and stored. The urokinase complex had a total activity of 21,000 IU when assayed on fibrin plate.

Example 4

0.1 ml urokinase (molecular weight 54,000, 1,000,000 IU/mol, phosphate buffer, pH 7.2) was mixed with 1 ml human plasma and incubated at 30°C for 30 min. for reaction. When the reaction was completed, the reaction solution was passed through a benzamidine succinyl-Sepharose affinity column (gel volume, 5ml) which had been equilibrated with 0.05 M Tris buffer containing 0.5 M sodium chloride at pH 8.0, adsorbing the unreacted urokinase. The column was washed with 10 ml of the buffer. The urokinase complex was recovered from a non-adsorbed fraction. The molecular weight of the urokinase complex was determined by electrophoresis to be about 91,000. The resulting urokinase complex had a total activity of 39,000 when assayed on fibrin plate.

Example 5

0.1 ml $^{14}$C-labelled urokinase (molecular weight 54,000, 37.7 µCi/76,000 IU/ml, 0.2% sodium chloride solution) labelled with $^{14}$C-acetate anhydride was mixed with 1 ml rat plasma, and incubated at 35°C for 15 min. for reaction. When the reaction was completed, the reaction solution was gel filtered over a Sephadex G-100 column (0.9 cm diameter×60 cm) in the same manner as in Example 1 to isolate $^{14}$C-labelled urokinase complex from unreacted $^{14}$C-labelled urokinase. The urokinase complex was determined by gel filtration to have a molecular weight of approximately 94,000. The resulting urokinase complex was frozen at −80°C and stored. Emulsion scintillator was added to a portion of the $^{14}$C-labelled urokinase to measure radioactivity. The total radioactivity was 1.24 µCi.

The parameters used for the preparation of urokinase complexes in these examples are summarized in Table 1 along with the results.

TABLE 1
Preparation of urokinase complex

| Example | Starting materials | Method for isolation | Total urokinase activity |
|---------|--------------------|----------------------|--------------------------|
| 1 | urokinase+ rat plasma | gel filtration (Sephadex G-100) | 6,300 IU |
| 2 | urokinase+ rabbit serum | SDS-polyacrylamide slab gel electrophoresis, protein recovery | 7,500 IU |
| 3 | urokinase+ beagle whole blood | centrifuge, preparative gel electrophoresis | 21,000 IU |
| 4 | urokinase+ human plasma | affinity chromatography (benzamidine succinyl-Sepharose) | 39,000 IU |
| 5 | $^{14}$C-labelled urokinase+rat plasma | gel filtration (Sephadex G-100) | 1.24 µCi (total radioactivity) |

The thus obtained urokinase complexes were subjected to the following experiments to ascertain their effectiveness.

Experiment 1: Affinity to fibrin

$^{14}$C-labelled urokinase and $^{14}$C-labelled urokinase complex prepared in Example 5 were compared with respect to affinity to fibrin.

(i) Solutions of $^{14}$C-labelled urokinase and $^{14}$C-labelled urokinase complex were prepared at concentrations of 0.002 and 0.004 µCi/ml (corresponding to 10 and 20 IU/ml of $^{14}$C-labelled urokinase). Control solutions of standard urokinase (Japanese standard available from the Japan National Institute of Health) were also prepared at concentrations of 10 and 20 IU/ml. 10 µl of each solution was spotted on a fibrin plate and incubated at 37°C for 16 hours. The lysis window or area appearing on the fibrin plate was determined in terms of its major and minor diameters in mm. The results are shown in Table 2.

The lysis areas by $^{14}$C-labelled urokinase were substantially equal to those by the standard urokinase. The lysis areas by $^{14}$C-labelled urokinase complex according to the invention were larger than those by $^{14}$C-labelled urokinase and standard urokinase. Although these urokinase compounds were spotted on fibrin plates in an equivalent amount as urokinase activity, the instant urokinase complex exhibited a larger lysis area than $^{14}$C-labelled urokinase or standard urokinase, demonstrating that the urokinase complex has a great affinity to fibrin.

TABLE 2
Affinity to fibrin

| Sample | Concentration, IU/ml | Lysis area, mm |
|---|---|---|
| Standard urokinase | 10 | 14.3×14.2 |
| | 20 | 16.3×15.9 |
| $^{14}$C-labelled urokinase | 10 | 14.3×14.0 |
| | 20 | 16.0×15.8 |
| $^{14}$C-labelled urokinase complex | 10 | 15.6×15.7 |
| | 20 | 18.5×18.3 |

(ii) 10,000 IU $^{14}$C-labelled urokinase was mixed with 10,000 IU $^{14}$C-labelled urokinase complex. The mixture was applied to a fibrin-Sepharose® affinity column (gel volume, 5 ml) which had been equilibrated with 0.01 M phosphate buffer at pH 7.6. The column was first washed with 30 ml of 0.01 M phosphate buffer containing 1 mM EDTA (ethylenediaminetetraacetic acid) and 0.5 M sodium chloride at pH 7.6. The protein adsorbed in the column was then eluted with 0.01 M phosphate buffer containing 1 mM EDTA and 0.2 M arginin at pH 7.6. Aliquots of the wash and elute were subjected to SDS-polyacrylamide slab gel electrophoresis along with $^{14}$C-labelled urokinase and $^{14}$C-labelled urokinase complex, and then analysed by the fibrin plate method as in Example 2. Each solution was thus determined for fibrinolytic activity and radioactivity.

Analysis of the wash displayed lysis window and radio activity at a position substantially equivalent to $^{14}$C-labelled urokinase. Analysis of the elute displayed lysis window and radioactivity at a position substantially equivalent to $^{14}$C-labelled urokinase complex.

The results indicate that urokinase complex was adsorbed by fibrin while urokinase was not adsorbed by fibrin, proving the increased affinity of the urokinase complex to fibrin.

Experiment 2: Retention of blood

The residual urokinase activity in blood of both urokinase and the urokinase complex prepared in Example 1 was determined by intravenously injecting them to rats to measure theiv half time in blood.

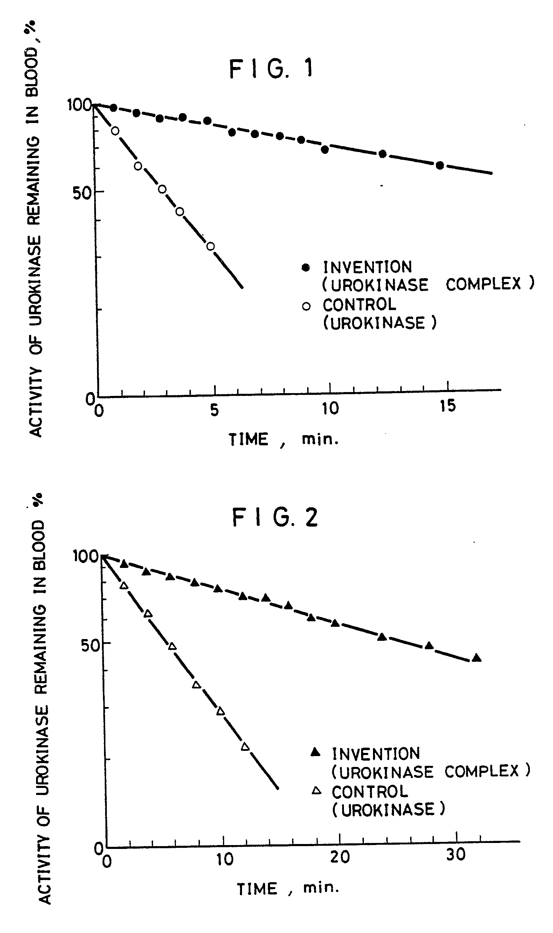

0.5 ml saline portions containing 50,000 IU of urokinase and urokinase complex were respectively administered to male Wistar rats weighing 200—300 grams through their tail vein. Blood was collected from the inferior vena cava at regular time intervals after administration. A 3.8% citrate solution having 500 KIE/ml aprotinin added thereto was added to the collected blood in an amount of 10% by volume. The citrated blood was centrifuged at 4°C, 3,000 rpm for 10 minutes to separate the plasma. The urokinase activity in plasma was measured using a synthetic substrate S-2444. The results are shown in Fig. 1.

The conventional or as-isolated urokinase had a half time of about 3 minutes whereas the urokinase complex of the invention had a half time of about 18 minutes which proved the improved retention in blood.

The urokinase activity shown in Fig. 1 is an average of measurements using 5 rats in each set.

Experiment 3: Retention in blood

The residual urokinase activity in blood of both urokinase and urokinase complex of the invention was determined by intravenously injecting them to rabbits to measure their half time in blood.

4 ml saline portions containing 30,000 IU of urokinase and urokinase complex were respectively adminstered to male rabbits weighing approximately 2 kg through their auricular vein. Blood was collected through a catheter retained in the right femoral vein. The procedure of Experiment 2 was then repeated. The results are shown in Fig. 2.

The conventional urokinase had a half time of about 5.5 minutes whereas the urokinase complex of the invention had a half time of about 25 minutes which proved the improved retention in blood.

The urokinase activity shown in Fig. 2 is an average of measurements using 3 rabbits in each set.

Experiment 4: Retention in blood

The residual urokinase activity in blood of both urokinase and urokinase complex of the invention was determined by intravenously injecting them to beagles to measure their half time in blood.

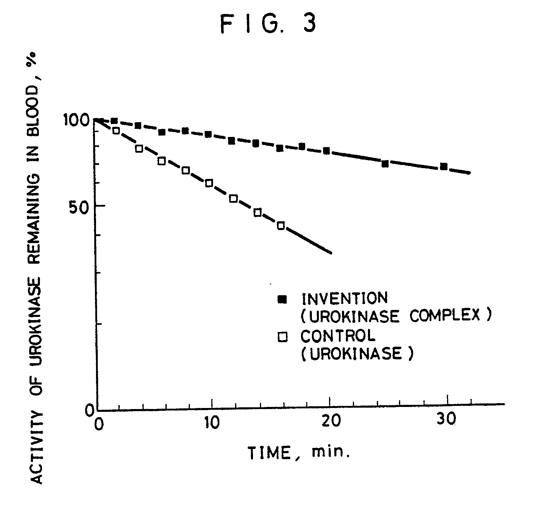

5 ml saline portions containing 60,000 IU of urokinase and urokinase complex were respectively administered to female beagles weighing approximately 10 kg through the vein in their right hind leg. Blood was collected from the vein in the fore leg. The procedure of Experiment 2 was then repeated. The results are shown in Fig. 3.

The conventional urokinase had a half time of about 13 minutes whereas the urokinase complex of the invention had a half time of about 45 minutes which proved the improved retention of the urokinase complex of the present invention. The urokinase activity shown in Fig. 3 is an average of measurements using 3 beagles in each set.

Effects of the invention

As evident from the examples and experiments, the urokinase complex according to the present invention has a high affinity to fibrin, a long half time in blood, and a remarkably prolonged retention of fibrinolytic activity in blood by a factor of 3 or more as compared to urokinase alone.

The urokinase complex can be readily prepared by mixing urokinase with whole blood, plasma or serum.

The urokinase complex of the present invention may also be contained in a fibrinolytic agent. Since the urokinase complex has a high binding affinity to fibrin and prolonged fibrinolytic activity, the resulting fibrinolytic agent is fully active in minor quantities.

## Claims

1. Process for preparing a urokinase complex, comprising mixing urokinase with whole blood, blood serum or blood plasma allowing the mixture to react with the serum protein or plasma protein to form a urokinase complex fraction and separating and purifying said urokinase complex fraction having a molecular weight of 80,000 to 120,000 from the reaction mixture.

2. A process according to claim 1, wherein the urokinase complex is an integral complex in which urokinase is bound to protein in the blood component.

3. A process according to claim 1, wherein the mixture is allowed to react at 4°C to 40°C for 15 to 30 minutes.

4. A urokinase complex prepared by a process according to claim 1.

5. A pharmaceutical composition containing a urokinase complex according to claim 4 and conventional vehicles.

## Patentansprüche

1. Verfahren nach Herstellung eines Urokinase-Komplexes durch Vermischen von Urokinase mit Vollblut, Blutserum oder Blutplasma, Reagierenlassen des Gemischs mit dem Serumprotein oder Plasmaprotein zur Bildung einer Urokinase-Komplexfraktion sowie Abtrennen und Reinigen der Urokinase-Komplexfraktion eines Moleukargewichts von 80 000 bis 120 000 aus dem Reaktionsgemisch.

2. Verfahren nach Anspruch 1, dadurch gekennzeichnet, daß es sich bei dem Urokinase-Komplex um einen integralen Komplex, in dem Urokonase an das Protein in der Blutkomponente gebunden ist, handelt.

3. Verfahren nach Anspruch 1, dadurch gekennzeichnet, daß das Gemisch während 15 bis 30 min bei 4°C bis 40°C reagieren gelassen wird.

4. Urokinase-Komplex, hergestellt nach einem Verfahren gemäß Anspruch 1.

5. Arzneimittel mit einem Urokinase-Komplex nach Anspruch 4 und üblichen Trägern.

## Revendications

1. Procédé de préparation d'un complexe d'urokinase, comprenant le mélange d'urokinase avec le sang complet, le sérum sanguin ou le plasma sanguin, le fait de laisser le mélange réagir avec une protéine du sérum ou une protéine du plasma pour former une fraction complexe d'urokinase, et la séparation et la purification, à partir du mélange réactionnel, de cette fraction complexe d'urokinase, qui a un poids moléculaire de 80.000 à 120.000.

2. Procédé selon la revendication 1, dans lequel le complexe d'urokinase est un complexe intégral dans lequel l'urokinase est liée à une protéine de composant du sang.

3. Procédé selon la revendication 1, dans lequel on laisse réagir le mélange entre 4°C et 40°C pendant 15 à 30 minutes.

4. Complexe d'urokinase préparé à l'aide d'un procédé selon la revendication 1.

5. Composition pharmaceutique contenant un complexe d'urokinase selon la revendication 4, et des véhicules classiques.

# F I G. 1

ACTIVITY OF UROKINASE REMAINING IN BLOOD , %

TIME , min.

● INVENTION
(UROKINASE COMPLEX )
○ CONTROL
(UROKINASE )

# F I G. 2

ACTIVITY OF UROKINASE REMAINING IN BLOOD %

TIME , min.

▲ INVENTION
(UROKINASE COMPLEX )
△ CONTROL
(UROKINASE )

1

## F I G. 3